# EUROPÄISCHE PATENTSCHRIFT

(11) **EP 0 603 627 B1**
(45) Veröffentlichungstag und Bekanntmachung des Hinweises auf die Patenterteilung: **14.02.1996**
(21) Anmeldenummer: 93119650.5
(22) Anmeldetag: 06.12.1993
(51) Int. Cl.: C01G 9/02, A61K 7/42

(54) **Feinteiliges, hochreines, neutrales Zinkoxidpulver, Verfahren zu dessen Herstellung und seine Verwendung**
Fine, highly pure, neutral zinc oxide, process for its preparation and use thereof
Poudre d'oxyde de zinc neutre très pure finement divisée, procédé de sa préparation et son utilisation

(30) Priorität: 18.12.1992 DE 4242949
(43) Veröffentlichungstag der Anmeldung: 29.06.1994
(73) Patentinhaber: BAYER AG, D-51368 Leverkusen (DE); HAARMANN & REIMER GMBH, D-37601 Holzminden (DE)
(72) Erfinder: Miksits, Michael, Dr., D-42279 Duisburg (DE); Tiburtius, Christoph, Dr., D-50999 Köln (DE); Kischkewitz, Jürgen, Dr., D-40883 Ratingen (DE); Bütje, Kai, Dr., D-47229 Duisburg (DE); Warth, Albrecht, D-45478 Mülheim/Ruhr (DE); Herzig, Franz, D-47809 Krefeld (DE); Langner, Roland, D-37639 Bevem (DE)
(74) Vertreter: Müller, Gerhard, Dr.

(56) Entgegenhaltungen:
- DE-C- 825 543
- US-A- 2 144 299
- PATENT ABSTRACTS OF JAPAN vol. 4, no. 77 (C-13) (559) 4. Juni 1980 & JP-A-55 042 282 (DOUWA KOGYO K.K.) 25. März 1980
- DATABASE WPI Week 9246, Derwent Publications Ltd., London, GB; AN 92-378833 & JP-A-4 280 814 (HAYASHI KINZOKU KOGYOSHO KK) 6. Oktober 1992
- DATABASE WPI Week 8306, Derwent Publications Ltd., London, GB; AN 83-13189K & JP-A-57 209 824 (MITSUBISHI METAL KK) 23. Dezember 1982

## Beschreibung

Die Erfindung betrifft feinteiliges, hochreines, neutrales Zinkoxidpulver, das für den UV-Schutz in kosmetischen Sonnenschutz-Zubereitungen und Tageshautpflegepräparaten sowie in Lacken und Kunststoffen Verwendung findet, sowie ein Verfahren zu dessen Herstellung.

Seit einigen Jahren setzt sich in zunehmendem Maße die Erkenntnis durch, daß der ultraviolette Strahlungsanteil des Sonnenlichtes auf ungeschützter Haut Schädigungen hervorrufen kann, die vom akuten Sonnenbrand (Erythem) über vorzeitige Alterungserscheinungen bis zum Hautkrebs reichen können. Für die akuten Schädigungen wird in erster Linie der relativ kurzwellige, gewöhnlich als UV-B bezeichnete Anteil zwischen ca. 280 und 320nm verantwortlich gemacht, für die vorzeitige Hautalterung dagegen besonders der als UV-A bezeichnete längerwellige Anteil zwischen ca. 320 und 400 nm.

Sonnenschutz-Zubereitungen und Tageshautpflegeprodukte mit UV-Schutz schützen die Haut vor einem oder beiden der ultravioletten Strahlungsanteile durch Absorption und/oder Reflexion (Streuung). Für den UV-B-Bereich existiert dabei eine breite Palette organischer Verbindungen, die gewöhnlich als UV-Filter bezeichnet und in großem Ausmaß eingesetzt werden. Die Auswahl an Filtern für den UV-A-Bereich ist im Vergleich dazu sehr begrenzt. Die hierfür verwendeten organischen Substanzen können zum Teil beträchtliche Probleme hinsichtlich ihrer Löslichkeit, Stabilität und Hautverträglichkeit aufweisen.

Als Ausweg aus dieser Situation werden seit einigen Jahren in rasch steigendem Ausmaß feinteilige anorganische Pigmente als Absorber und Streuer im UV-ABereich eingesetzt. Aufgrund der Lage ihrer Absorptionskanten kommen insbesondere Titandioxid in der Rutilmodifikation und Zinkoxid in Betracht, wobei letzteres aufgrund seiner besseren Dispergierbarkeitseigenschaften, die im Gegensatz zu TiO₂ zu im sichtbaren Spektralbereich transparenten Schichten führen, dem ersteren überlegen ist.

Zinkoxidpulver kann für sich oder in Kombination mit UV-B-Filtern eingesetzt werden, woraus ein sogenannter UV-Breitbandschutz resultiert. Dies ist aus der Literatur bekannt.

Beispielsweise offenbart JP 60/231 607 Sonnenschutz-Zubereitungen, die als wirksamen Bestandteil 1 bis 30 Gew.-% feinteiliges ZnO mit einem maximalen Teilchendurchmesser <0,1 µm und einem durchschnittlichen Durchmesser von 10 bis 60 nm enthalten. In DE 3 642 794 sind Zubereitungen mit 1 bis 25 Gew.-% Zinkoxid mit einer durchschnittlichen Teilchengröße zwischen 70 und 300 nm beschrieben. Die Anmeldung JP 62/084 017 offenbart Kosmetika, die 0,05 bis 30Gew.-% transparentes Zinkoxid mit Teilchengrößen < 300 Å enthalten.

Ferner ist bekannt, anorganisch nachbehandeltes Zinkoxid einzusetzen, wie in JP 03/183 620 beschrieben, sowie Zinkoxid in Kombination mit UV-B-Filtern aus den Reihen der Zimtsäureester (DE 2 533 497), der Dibenzoylmethan- und der Paraaminobenzoesäurederivate (JP 61/215 314 und JP 61/257 915) oder mit TiO₂ (EP 433 086; Sonnenschutzmittel mit je 2 bis 25 Gew.-% ZnO und Rutil-TiO₂). Auch im Gemisch mit organischen hochmolekularen Pulvern oder als Coating auf solchen Pulvern kann ZnO verwendet werden, wie beispielsweise in JP 03/200 721 und JP 02/049 717 beschrieben.

Feinteiliges Zinkoxid für den kosmetischen Bereich muß verschiedene Anforderungen erfüllen. Zunächst muß es ausreichend Schutz vor UV-Strahlung durch UV-Absorption bieten und gleichzeitig im sichtbaren Bereich möglichst transparent sein, um keinen kosmetisch unerwünschten weißen Film auf der Haut zu hinterlassen. Für einen optimalen Schutz gibt es ein Optimum in der Teilchengröße, wie beispielsweise in DE 3 642 794 beschrieben. Ferner darf, um den Anforderungen der gesundheitlichen Tests zu genügen, ein bestimmter Gehalt an Schwermetallionen nicht überschritten werden.

Von den drei in der Literatur beschriebenen technischen Verfahren zur Herstellung von Zinkoxid, dem sogenannten französischen, amerikanischen und dem naßchemischen Prozeß (Ullmanns Enzyklopädie der technischen Chemie, 4. Auflage, Band 24, Seiten 635 f.), ist insbesondere der letztere zur Herstellung von feinteiligem Zinkoxid geeignet. Zwar ist in JP 01/286 919 ein trockener Prozeß vorgeschlagen worden, der in der Lage ist, feinteiliges ZnO mit einer spezifischen Oberfläche von z.B. 25 m/g zu liefem, jedoch setzt dieser eine aufwendige Reinigung der Ausgangsstoffe voraus, wenn unakzeptabel hohe Schwermetallgehalte im Endprodukt vermieden werden sollen.

Das naßchemische Verfahren besteht im Prinzip in einer Umsetzung einer Zinksalzlösung mit einem Überschuß einer wäßrigen Alkali- oder Ammoniumhydroxid- oder -carbonatlösung, wodurch ein Zinkhydroxid oder ein basisches Zinkcarbonat ausgefällt wird, welches durch eine Hitzebehandlung (Kalzination) in Zinkoxid überführt wird.

Beispiele für derartige Verfahren sind in den deutschen Patentschriften 2 404 049, 825 543, 744 937, 527 167, 481 284 und in US 2 144 299 beschrieben. Diese Beispiele zeigen aber auch, daß es zum Erreichen eines feinteiligen, oberflächenreichen, hochreinen Zinkoxids unerläßlich ist, ganz bestimmte Bedingungen einzuhalten.

Andere Prozesse zur Herstellung von hochreinem, feinteiligem ZnO, wie die in JP 02/129 135 und WO 92/13517 beschriebenen, die von Zink-Alkoxiden oder Zink-Alkylen ausgehen, benutzen teure Ausgangsmaterialien und sind daher unwirtschaftlich. Ebenso läßt die in JP 57/205 319, JP 57/209 824 und JP 03/050 119 vorgeschlagene Fällung und Zersetzung von Zinkoxalat keine spezifischen Vorteile gegenüber der Carbonatfällung erkennen.

Den bisherigen Zubereitungen haftet jedoch der Nachteil an, daß bei ihrer Anwendung die Haut zum Teil angegriffen wird, und daß das Zinkoxid zu hohe Anteile an Fremdmetallionen enthält. Außerdem sind die Zubereitungen teilweise nicht ausreichend stabil.

Aufgabe war es daher, ein feinteiliges, oberflächenreiches Zinkoxidpulver zur Verfügung zu stellen, das keine oder kaum Fremdmetallionen enthält, neutral reagiert und das es gestattet, bei Anwendung in kosmetischen Zubereitungen keine Reizungen der Haut zu verursachen und stabile Zubereitungen zu liefern, sowie ein Verfahren zu dessen Herstellung.

Gegenstand der Erfindung ist ein feinteiliges, hochreines, neutrales Zinkoxidpulver für UV-Schutz, welches dadurch gekennzeichnet ist, daß es eine spezifische BET-Oberfläche zwischen 30 und 100 m/g, einen pH-Wert zwischen 6,0 und 7,6 gemäß DIN ISO 787, Teil 9 sowie einen besonders niedrigen Schwermetallgehalt von jeweils weniger als 5 ppm der Elemente Co, Ni, Pb, Cd, Cu, Mn, Fe und Hg aufweist.

Die Bestimmung der spezifischen Oberfläche erfolgt nach der BET-Methode (DIN 66 131; siehe auch F.M. Nelsen,F.T. Eggertsen, Analyt. Chem. 30 (1958), 1387 oder S.J. Gregg, K.S.W. Sing, Adsorption, Surface Area and Porosity, London New York 1967, Chapt. 2,8; Adsorption von Stickstoff bei 77 K).

Üblicherweise liegt der mittlere Primärteilchendurchmesser des Zinkoxidpulvers zwischen 5 und 100 nm.

Bevorzugt liegt das Zinkoxidpulver mit einer anorganischen Nachbehandlungsschicht bestehend aus einem oder mehreren Hydroxiden, Oxidhydraten oder Oxiden der Elemente Aluminium, Silicium, Zirkonium oder Titan in einer Menge von 0,5 bis 15 Gew.-%, bezogen auf ZnO, vor.

Besonders bevorzugt ist das Zinkoxidpulver hydrophob. Als Hydrophobierungsmittel werden vorzugsweise Silikonöle eingesetzt.

Ein weiterer Gegenstand der Erfindung sind stabile Dispersionen von Zinkoxidpulver in einer wäßrigen oder einer Ölphase, die ausgewählt ist aus den Gruppen mineralischer, pflanzlicher oder tierischer Öle oder langkettiger Kohlenwasserstoffverbindungen oder Ester, welche dadurch gekennzeichnet sind, daß sie 0,1 bis 50 Gew.-% des erfindungsgemäßen Zinkoxidpulvers enthalten.

Ein weiterer Gegenstand der Erfindung sind kosmetische Zubereitungen, wie beispielsweise Sonnenschutzcremes und -lotionen, Tageshautpflegecremes und -lotionen mit UV-Schutz, welche dadurch gekennzeichnet sind, daß sie 0,5 bis 20 Gew.-%, bevorzugt 0,5 bis 5 Gew.-%, des erfindungsgemäßen Zinkoxidpulvers entweder allein oder in Kombination mit einem oder mehreren anorganischen und/oder organischen UV-Schutz-Wirkstoffen enthalten.

Ein weiterer Gegenstand der Erfindung ist ein Verfahren zur Herstellung des erfindungsgemäßen Zinkoxidpulvers durch Fällung von basischem Zinkcarbonat aus gegebenenfalls vorgereinigten Zinksulfat- und/oder Zinkchloridlösungen mittels Alkalicarbonatlösungen, welches dadurch gekennzeichnet ist, daß die Fällung diskontinuierlich unter Vorlage der Zinksalzlösung und Zugabe der Alkalicarbonat-Lösung oder kontinuierlich durch gleichzeitige Zugabe von Zinksalz- und Alkalicarbonat-Lösung in einem pH-Bereich von 5,2 bis 6,5, vorzugsweise 5,8 bis 6,3, durchgeführt wird, und daß das Fällprodukt von der Mutterlauge abgetrennt, gegebenenfalls gewaschen, kalziniert und gemahlen wird.

Bevorzugt wird das Filtrat nach der Fällung durch Zugabe weiterer Alkalicarbonat-Lösung bei einem pH-Wert oberhalb 6,5 aufgearbeitet und das Fällprodukt dieser zweiten Fällung nach Abtrennung von der Mutterlauge und Wiederauflösung in Säure in den Prozeß zurückgeführt.

Die Fälltemperatur liegt vorzugsweise bei 50 bis 90°C, besonders bevorzugt bei 60 bis 80°C.

Vorzugsweise enthalten die eingesetzten Lösungen 5 bis 20 Gew.-% Alkalicarbonat und 30 bis 130 g Zink/l. Als Alkalicarbonatlösung wird bevorzugt Sodalösung eingesetzt.

Besonders bevorzugt wird das Zinkoxid im Anschluß an die Kalzination einer anorganischen Nachbehandlung unterworfen.

Im Anschluß an die Kalzination oder im Anschluß an die anorganische Nachbehandlung kann das Zinkoxid noch hydrophobiert werden. Als Hydrophobierungsmittel werden bevorzugt Silikonöle eingesetzt.

Das erfindungsgemäße Zinkoxidpulver wird als UV-Schutzkomponente in kosmetischen Zubereitungen, insbesondere in Sonnenschutz-Zubereitungen verwendet.

Das erfindungsgemäße Zinkoxidpulver wird auch in Kunststoffen und Lacken als UV-Schutzkomponente verwendet.

Marktübliches feinteiliges Zinkoxid, das nach dem bekannten, naßchemischen Verfahren hergestellt wird, enthält üblicherweise Verunreinigungen in Form von restlichen Fremdmetalloxiden und/oder freiem Carbonat. Diese Verunreinigungen können zu einer alkalischen Reaktion führen. Eine solche alkalische Reaktion beeinflußt die Eigenschaften des Zinkoxids in der kosmetischen Zubereitung negativ. Eine alkalische Reaktion ist in Sonnenschutz- und Tageshautpflegeprodukten unerwünscht, da zum einen Hautreizungen auftreten können und zum anderen die Stabilität der Zubereitung in hohem Maße beeinträchtigt wird.

Zwar ist es möglich, den alkalischen pH-Wert der Formulierung durch die Zugabe sauer reagierender Komponenten zu erniedrigen, jedoch geht dieser Effekt sehr häufig mit einer deutlichen Verminderung der Stabilität der Emulsionen einher. Auch Versuche, die Oberfläche von handelsüblichem, alkalisch reagierendem, feinteiligem Zinkoxid organisch zu beschichten, beispielsweise mit einem hydrophobierenden Silikonöl, führten nicht zu der gewünschten Stabilisierung des pH-Wertes im neutralen Bereich.

Auch bei der Anwendung von Zinkoxid in Lacken und Kunststoffen, denen Zinkoxid als UV-Schutzkomponente beigefügt wird, kann der pH-Wert eine Rolle spielen, wenn die diesen Lacken und Kunststoffen zugrundeliegenden Polymere durch alkalisch reagierende Komponenten abgebaut oder zerstört werden.

Durch die aus der Literatur bekannten Verfahren konnte bisher kein Zinkoxidpulver hergestellt werden, das gleichzeitig alle Anforderungen bezüglich Feinteiligkeit, Reinheit und neutraler Reaktion sowie wirtschaftlicher Prozeßführung erfüllt.

In DE 2 404 049 und DE 744 937 werden kontinuierliche Prozesse bei Fällungs-pH-Werten zwischen 7 und 10 bzw. 6,5 und 8 beschrieben, bei denen es nicht gelingt, ein neutral reagierendes Endprodukt zu erhalten.

Nach DE 825 543 wird aus stark verdünnten, in einem Überschuß von 2 bis 5 % vorliegenden ZnSO₄-Lösungen basisches Zinkcarbonat mit Sodalösung bei 35 bis 45°C ausgefällt. Die starke Verdünnung macht das Verfahren unwirtschaftlich, zudem ist der ausgefällte, basische Zinkcarbonat-Niederschlag schwer filtrier- und waschbar und üblicherweise nicht genügend rein.

Nach DE 527 167 wird eine maximal 1,5 normale Carbonatlösung mit einem geringen Überschuß einer maximal 1,5 normalen Zinksalzlösung versetzt. Auch hier führt die niedrige Konzentration zu niedrigen Raum-Zeit-Ausbeuten und verursacht Abwasserprobleme. Außerdem enthält der Niederschlag alkalisch reagierende Verunreinigungen.

In DE 481 284 und US 2 144 299 wird zur Reinigung des basischen Zinkcarbonatniederschlages eine Behandlung mit Kohlensäure bzw. Chlorid-, Nitrat-oder Sulfatlösungen vorgeschlagen. Dadurch werden zusätzlich Fremdionen eingeschleust und das Verfahren wird technisch sehr aufwendig.

Nach JP 03/199 121 wird ein feinteiliges Zinkoxidpulver durch Zugabe einer Zinksalzlösung zu einer wäßrigen Ammoniumcarbonat- oder -hydrogencarbonatlösung und Kalzination des Niederschlages erhalten. Abgesehen vom im Vergleich zu Natriumcarbonat höheren Preis der zur Fällung benutzten Ammoniumcarbonate fällt bei diesem Verfahren als Nebenprodukt eine Ammoniumsalzlösung an, deren umweltgerechte Entsorgung einen beträchtlichen Aufwand erfordert.

Gemäß JP 04/164 813, 04/164 814, 04/164 815 und 04/164 816 wird feinteiliges Zinkoxid durch Umsetzung von Zinksalzlösungen mit Alkalilaugen bei Temperaturen oberhalb 60°C und pH-Werten oberhalb 9 erhalten. Solche Zinkoxidpulver reagieren jedoch stets alkalisch.

Bei dem erfindungsgemäßen Verfahren kann der Zinkfällung eine bereits in US 2 402 371 beschriebene Reinigungsstufe vorausgehen, die darin besteht, daß die oxidierbaren Schwermetallionen Mn⁺ und Fe⁺ durch eine Behandlung mit Chlor und Natronlauge in höhere Wertigkeitsstufen überführt und gemeinsam in Form ihrer Oxidhydrate ausgefällt und abgetrennt werden. Durch Zementation mit Zinkmetall können unerwünschte Schwermetallionen (Co⁺, Ni⁺, Pb⁺, Cu⁺, Cd⁺ und Hg⁺) ebenfalls ausgefällt und abgetrennt werden.

Ein wesentliches Merkmal des erfindungsgemäßen Verfahrens ist, daß die Fällung der Gesamtmenge des Zinks zweistufig erfolgt, wobei in der ersten Stufe die Umsetzung der gegebenenfalls vorgereinigten Zinksalzlösung mit der Carbonatlösung bei einem pH-Wert von 5,2 bis 6,5, vorzugsweise 5,8 bis 6,3, durchgeführt wird.

Überraschend wurde gefunden, daß nur in diesem sehr engen pH-Wert-Bereich ein Fällprodukt erhalten wird, welches nach der üblichen Weiterverarbeitung ein neutrales Zinkoxid mit pH-Werten zwischen 6,0 und 7,6 gemäß DIN ISO 787, Teil 9 ergibt. Es ist entscheidend, daß der erfindungsgemäße pH-Wert-Bereich zu keinem Zeitpunkt der Fällung überschritten wird, weil sonst Zinkoxide mit zu hohen pH-Werten resultieren. Bei niedrigeren Fällungs-pH-Werten sind dagegen die Ausbeuten gering. Außerdem werden basische Zinksulfate und/oder -chloride mitgefällt.

Das Zinkcarbonat aus der ersten Fällstufe wird von der Mutterlauge erfindungsgemäß abgetrennt, z.B. durch Filtration, und gegebenenfalls nach Waschung durch Kalzination zu dem erfindungsgemäßen Zinkoxidpulver aufgearbeitet. Die Kalzination wird bevorzugt bei Temperaturen zwischen 350 und 500°C, besonders bevorzugt zwischen 400 und 450°C, durchgeführt, um ein feinteiliges, neutral reagierendes Zinkoxidpulver mit einer BET-Oberfläche zwischen 30 und 100 m/g zu erhalten.

Das Filtrat nach der ersten Fällung, welches noch Zink in Lösung enthält, kann mit weiterer Alkalicarbonatlösung im Überschuß versetzt werden, um bei pH-Werten oberhalb 6,5 eine zweite Fällung vorzunehmen. Hierbei ist es unerheblich, in welcher Reihenfolge die Lösung und das Fällungsreagens zusammengegeben werden und wo der genaue Fällungs-pH-Wert liegt. Das Produkt der zweiten Fällung wird vorzugsweise abfiltriert, in Säure aufgelöst und in den Prozeß zurückgeführt. Alternativ kann das Produkt der zweiten Fällung nach Filtration und Waschung kalziniert werden. Dieses erhaltene Zinkoxid zeigt jedoch nicht die erfindungsgemäßen Eigenschaften und kann daher auch nicht entsprechend der Erfindung verwendet werden.

Die Fällung des Zinkcarbonates in der ersten Fällungsstufe kann kontinuierlich oder diskontinuierlich vorgenommen werden. Bei einer kontinuierlichen Arbeitsweise werden Zinksalzlösung und Carbonatlösung gleichzeitig einem Reaktionsbehälter zugeführt, wobei die Mengendosierung vorteilhaft durch pH-Wert-Regelung erfolgt. Hierdurch, wie auch durch intensive Vermischung der Komponenten, kann sichergestellt werden, daß der erfindungsgemäße pH-Wert-Bereich bei der Fällung eingehalten wird.

Bei einer diskontinuierlichen Arbeitsweise kann grundsätzlich eine der Ausgangslösungen vorgelegt und die andere anschließend hinzugefügt werden. Das erfindungsgemäße Zinkoxid wird bei diskontinuierlicher Prozeßführung jedoch nur dann erhalten, wenn die Zinksalzlösung vorgelegt und die Carbonatlösung bis zum Erreichen des erfindungsgemäßen pH-Wertes zudosiert wird. Auch hier ist eine pH-Wert-Regelung unter intensiver Vermischung angebracht. Im umgekehrten Fall, d.h. bei Vorlage der Carbonatlösung und Zugabe von Zinksalzlösung, wird dagegen ein basisches Zinkcarbonat erhalten, das nach Aufarbeitung kein erfindungsgemäßes Zinkoxid ergibt.

Die Fällung des basischen Zinkcarbonates in der ersten Fällstufe wird vorzugsweise bei Temperaturen von 50 bis 90°C, besonders bevorzugt von 60 bis 80°C, durchgeführt. Unter diesen Bedingungen können auch höher konzentrierte Ausgangslösungen eingesetzt werden, ohne daß es zu unerwünschten Eindickungen im Reaktionsbehälter kommt. Es wird ein Fällungsprodukt erhalten, das sehr gut filtrier- und waschbar ist.

Die eingesetzten Lösungen enthalten im allgemeinen 5 bis 20 Gew.-% Alkalicarbonat bzw. 30 bis 130 g Zink/l. Aus wirtschaftlichen Gründen werden hoch konzentrierte Lösungen bevorzugt, wohingegen zwecks genauerer pH-Wert-Einstellung niedrig konzentrierte Lösungen vorzuziehen sind. Bevorzugt werden auch aus wirtschaftlichen Gründen Natriumcarbonat-(Soda-)Lösungen zum Fällen eingesetzt.

Das erfindungsgemäße Zinkoxidpulver kann vor der Einarbeitung in kosmetische Zubereitungen oder in Lacke oder Kunststoffe anorganisch und/oder organisch nachbehandelt werden, falls dies gewünscht wird. Eine anorganische Nachbehandlung kann beispielsweise in einer Auffällung einer Schicht eines oder mehrerer Oxide, Hydroxide oder Oxidhydrate der Elemente Silicium, Aluminium, Titan oder Zirkonium bestehen, wobei eine solche Nachbehandlungsschicht sinnvollerweise nicht weniger als 0,5 und nicht mehr als 15 Gew.-% solcher Oxide, Hydroxide oder Oxidhydrate enthalten soll.

Das Zinkoxidpulver oder das anorganisch nachbehandelte Pulver kann ferner mit einer organischen Nachbehandlungsschicht versehen werden, wobei insbesondere eine Behandlung mit einem Silikonöl zur Hydrophobierung und zur Verbesserung der Dispergierbarkeit in oleophilen Medien von Vorteil sein kann. Die Silikonisierung kann durch Besprühen mit einem geeigneten Silikonöl oder durch Suspendieren des Pulvers in der Lösung eines geeigneten Silikonöls in einem niedrig siedenden Lösungsmittel und Abdestillieren des Lösungsmittels erfolgen.

Die erfindungsgemäßen Dispersionen können neben dem erfindungsgemäßen Zinkoxidpulver noch weitere feinteilige Pigmente, wie z.B. Titandioxid, Füllstoffe oder organische UV-Filter enthalten.

Die erfindungsgemäßen kosmetischen Zubereitungen, wie beispielsweise Sonnenschutzcremes und -lotionen, Tageshautpflegecremes und -lotionen, die das feinteilige, neutral reagierende Zinkoxidpulver in einer Konzentration von 0,5 bis 20Gew.-%, bevorzugt 0,5 bis 5 Gew.-% enthalten, bieten der Haut einen guten Schutz vor UV-Strahlung und zeichnen sich gegenüber Zubereitungen unter Verwendung von herkömmlichen, alkalisch reagierendem Zinkoxid durch verbesserte Stabilität und Hautverträglichkeit aus. In diesen Zubereitungen kann das Zinkoxidpulver mit weiteren UV-Filtern oder auch anorganischen feinteiligen Pigmenten, z.B. Titandioxid, kombiniert werden.

Die Erfindung soll anhand der nachfolgenden Beispiele näher erläutert werden.

### Beispiele

### Beispiel 1

### Diskontinuierliche Herstellung des erfindungsgemäßen Zinkoxidpulvers

5 l einer durch Behandlung mit Natronlauge und Chlor gereinigten Zinksulfatlösung (97 g Zn/l) wurden mit derselben Menge destillierten Wassers verdünnt, auf 60°C erwärmt und binnen 20 Minuten unter Rühren und unter Aufrechterhaltung der Temperatur von 60°C mit 2,9 l einer Natriumcarbonatlösung (19 Gew.-% Na₂CO₃) versetzt. Der pH-Wert lag unmittelbar nach Ende der Fällung bei 5,9 und nach 30minütigem Nachrühren bei 6,0. Der Niederschlag aus basischem Zinkcarbonat wurde abfiltriert, gewaschen, getrocknet und 1 Stunde bei 430°C kalziniert. Es wurden 472 g eines neutralen Zinkoxidpulvers erhalten, dessen Eigenschaften in Tabelle 1 aufgeführt sind.

### Vergleichsbeispiel 1

### Diskontinuierliche Verfahrensweise

Es wurde wie in Beispiel 1 vorgegangen, jedoch wurde die Fällung bis zu einem pH-Wert von 6,7 unmittelbar nach dem Ende der Natriumcarbonatzugabe bzw. 6,8 nach 30minütigem Rühren durchgeführt. Es wurden 617 g eines alkalischen Zinkoxidpulvers erhalten, dessen Eigenschaften in Tabelle 1 aufgelistet sind.

### Beispiel 2

### Kontinuierliche Herstellung des erfindungsgemäßen Zinkoxidpulvers

Es wurde ähnlich wie in Beispiel 1 vorgegangen, wobei die verdünnte Zinksulfatlösung und die Natriumcarbonatlösung jedoch gleichzeitig mit einer solchen Geschwindigkeit in einen Reaktionsbehälter eindosiert wurden, daß der pH-Wert zwischen 6,2 und 6,3 bei 60°C lag. Die Eigenschaften des daraus erhaltenen Zinkoxidpulvers sind in Tabelle 1 aufgeführt.

### Vergleichsbeispiel 2

### Kontinuierliche Verfahrensweise

Es wurde wie in Beispiel 2 vorgegangen, mit dem Unterschied, daß die Fällung bei einem pH-Wert von 6,7 bis 6,8 vorgenommen wurde. Die Eigenschaften des daraus gewonnenen Zinkoxids können Tabelle 1 entnommen werden.

**Tabelle 1**

| | Gehalt ZnO % | Gehalt MgO % | Gehalt SO₄ ²⁻ % | pH-Wert | BET m/g |
|---|---|---|---|---|---|
| Beispiel 1 | 94,3 | 0,06 | 2,55 | 7,1 | 66 |
| Vergleichsbeispiel 1 | 93,0 | 1,11 | 1,30 | 9,7 | 67 |
| Beispiel 2 | 96,7 | 0,17 | 1,20 | 7,1 | 53 |
| Vergleichsbeispiel 2 | 95,2 | 0,79 | 0,13 | 10,3 | 54 |

### Beispiel 3

### Diskontinuierlicher Betriebsversuch

In einem zylindrischen Betriebskessel mit den Abmessungen ca. 4 m Durchmesser und ca. 3 m Höhe, der mit einem Propellerrührwerk ausgerüstet ist, wurden 4 m³ Zinksulfatlösung mit 95 g Zn/l vorgelegt und durch direkte Dampfeinleitung auf 60°C aufgeheizt. Dann wurde unter Rühren 8 %ige Sodalösung solange zudosiert, bis nach ca. 20 Minuten ein pH-Wert von 6,3 erreicht war. Die bis dahin zugegebene Sodalösung war. bezogen auf die Ausfällung von Zink als basisches Zinkcarbonat, unterstöchiometrisch, so daß ca. 1 g Zn/l ungefällt in Lösung blieb.

Das Fällungsprodukt wurde auf einem Drehfilter abgetrennt, der Filterkuchen dabei mit deionisiertem Wasser gewaschen, anschließend in einem weiteren Rührbehälter mit Wasser angeschlämmt und auf einem zweiten Drehfilter abfiltriert. Das so von löslichen Salzen wie Natrium- und Magnesiumsulfat befreite Fällprodukt wurde in üblicher Weise vorgetrocknet und in einem indirekt beheizten Drehrohrofen bei 400°C kalziniert.

In Tabelle 2 sind die Eigenschaften des erhaltenen Endproduktes aufgeführt.

Das Filtrat der Erstfiltration mit noch gelöstem Zink wurde in einem weiteren Rührbehälter mit einem Überschuß an 8 %iger Sodalösung versetzt bis zum Erreichen eines pH-Wertes von 8, wobei der Restgehalt an gelöstem Zink 5 mg/l betrug. Diese Suspension wurde dann über eine Filterpresse abfiltriert, der Filterkuchen abgetrennt, in Salzsäure aufgelöst und diese verdünnte Zinkchloridlösung zur Ausgangs-Zinksulfatlösung zurückgeführt.

**Tabelle 2**

| | | |
|---|---|---|
| ZnO | [%] | 96,8 |
| BET | [m/g] | 38 |
| pH-Wert | | 7,2 |
| MgO | [%] | 0,48 |
| SO₄ ²⁻ | [%] | 1,6 |
| Cl⁻ | [%] | 0,013 |
| Cu | [ppm] | < 1 |
| Pb | [ppm] | < 1 |
| Cd | [ppm] | < 1 |
| Mn | [ppm] | < 1 |
| Fe | [ppm] | < 1 |
| Co | [ppm] | < 1 |
| Ni | [ppm] | < 1 |
| Hg | [ppm] | <0,05 |
| As | [ppm] | <0,5 |
| Se | [ppm] | < 1 |

### Beispiel 4

### Einarbeitung des erfindungsgemäßen Zinkoxidpulvers in eine Emulsion

Das in Beispiel 1 hergestellte Zinkoxidpulver wurde in einer Gewichtsmenge von 5 % in eine Öl in Wasser-Emulsion⁽¹⁾ eingearbeitet. Nach mehrwöchiger Lagerung bei Raumtemperatur lag der pH-Wert der Emulsion bei 7,4. Dieser pH-Wert wird für kosmetische Hautschutzpflegemittel akzeptiert.

### ⁽¹⁾Öl in Wasser-Emulsion:

| Inhaltsstoffe/Handelsname | Lieferant * | CTFA**-Bezeichnung | % (W/W) |
|---|---|---|---|
| Teil A: | | | |
| Arlacel 165 | (1) | Glyceryl Stearate (and) PEG-100 Stearate | 4,00 |
| Eumulgin B 2 | (2) | Ceteareth-20 | 1,00 |
| Lanette O | (2) | Cetearyl Alcohol | 3,00 |
| Neo Heoliopan, Typ AV | (3) | Octyl Methoxycinnamate | 4,00 |
| Neo Heliopan, Typ E 1000 | (3) | Isoamyl p-Methoxycinnamate | 4,00 |
| Neo Heliopan Typ MBC | (3) | 4-Methylbenzylidene Camphor | 1,00 |
| Paraffinöl 65 cP | (4) | Mineral Oil | 2,00 |
| Myritol 318 | (2) | Caprylic/Capric Triglyceride | 5,00 |
| Abil 100 | (5) | Dimethicone | 1,00 |
| Zinkoxid | | Zinc Oxide | 5,00 |
| Solbrol P | (6) | Propylparaben | 0,08 |
| | | | |

| Teil B: | | | |
|---|---|---|---|
| Wasser, dest. | | Water | 64,92 |
| Veegum Ultra | (7) | Magnesium Aluminium Silicate | 1,50 |
| Glycerin | (2) | Glycerin | 1,50 |
| Sionit K flüssig | (6) | Sorbitol | 1,50 |
| Solbrol M | (6) | Methylparaben | 0,20 |
| | | | |

| Teil C: | | | |
|---|---|---|---|
| Parfumöl | (3) | Fragrance | 0,30 |

### Herstellungsvorschrift:

### Teil A:

Außer Zinkoxid werden alle Inhaltsstoffe in einen Rührkessel eingewogen und unter Rühren auf 70 bis 75°C erhitzt. Dann wird Zinkoxid zugegeben und Teil A ca. eine Minute homogenisiert.

### Teil B:

Das Wasser wird nach Zugabe von Solbrol M in einem separaten Kessel auf ca. 90°C erhitzt. Dann wird Veegum Ultra zugegeben und eine Dispergierung mit dem Ultra Turax durchgeführt. Anschließend werden Glycerin und Sionit K eingerührt. Dann wird unter Rühren Teil B in Teil A eingerührt.

### Teil C:

Nachdem die Emulsion auf ca. 40°C abgekühlt ist, wird das Parfumöl zugegeben und die Emulsion auf Raumtemperatur abgekühlt.

### *Lieferant:

(1) ICI Speciality Chemicals, Goldschmidtstr. 100, D-4300 Essen 1
(2) Henkel KGaA, Dehydag Cospha, Postfach 11 00, D-4000 Düsseldorf 1
(3) Haarmann & Reimer GmbH, Postfach 12 53, D-3450 Holzminden
(4) Hansen & Rosenthal, Heilholtkamp 11, D-2000 Hamburg 60
(5) Th. Goldschmidt AG, Goldschmidtstr. 100, D-4300 Essen 1
(6) Bayer AG, D-5090 Leverkusen, Bayerwerk
(7) Erbslöh, Kajen 12, D-2000 Hamburg 11

### **CTFA:

CTFA International Cosmetic Ingredient Dictionary, Fourth Edition
- Herausgegeben durch:: The Cosmetic, Toiletry and Fragrance Association, 1101 17th Street, N.W. Suite 300, Washington. D.C. 20036

### Vergleichsbeispiel 3

### Einarbeitung eines ZnO-Pulvers in eine Emulsion

Es wurde wie in Beispiel 4 vorgegangen, jedoch wurde anstelle des erfindungsgemäßen Zinkoxidpulvers aus Beispiel 1 das stark alkalisch reagierende Zinkoxid aus Vergleichsbeispiel 1 verarbeitet. Nach mehrwöchiger Lagerung bei Raumtemperatur lag der pH-Wert der Emulsion bei 9,0. Dieser pH-Wert liegt für kosmetische Hautschutzpflegemittel zu hoch und wird nicht akzeptiert.

### Vergleichsbeispiel 4

### Einarbeitung eines ZnO-Pulvers in eine Emulsion

Es wurde wie in Beispiel 4 vorgegangen. jedoch wurde anstelle des erfindungsgemäßen Zinkoxidpulvers aus Beispiel 1 ein durch herkömmliche Fällung (Vorlage von Natriumcarbonatlösung, Zugabe der Zinksulfatlösung, Filtration, Wäsche und Kalzinierung) erhaltenes Zinkoxid mit einem pH-Wert von ca. 10,5 (nach DIN ISO 787, Teil 9) und einer spezifischen Oberfläche von ca. 50 m/g verwendet. Nach mehrwöchiger Lagerung bei Raumtemperatur lag der pH-Wert der Emulsion bei 9,4. Dieser pH-Wert ist für kosmetische Hautschutzpflegemittel zu hoch und wird nicht akzeptiert.

### Beispiel 5

Das erfindungsgemäße Zinkoxidpulver aus Beispiel 3 wurde durch zweistündiges Dispergieren auf einem Skandex-Farbmischer mit Glasperlen in einer Konzentration von 10Gew.-% unter Zusatz von 0,5 Gew.-% Sojalecithin in Isopropylpalmitat eingearbeitet. Die Messung des UV-Vis-Transmissionsspektrums erfolgte in einer Quarzküvette mit 10µm Schichtdicke in Kugelgeometrie (RSA-PE-20-Kugel von Labsphere) mit einem Perkin Elmer Lambda 2-Spektralphotometer. Kalibriert wurde gegen Isopropylpalmitat. Das Spektrum ist in der Fig. wiedergegeben.

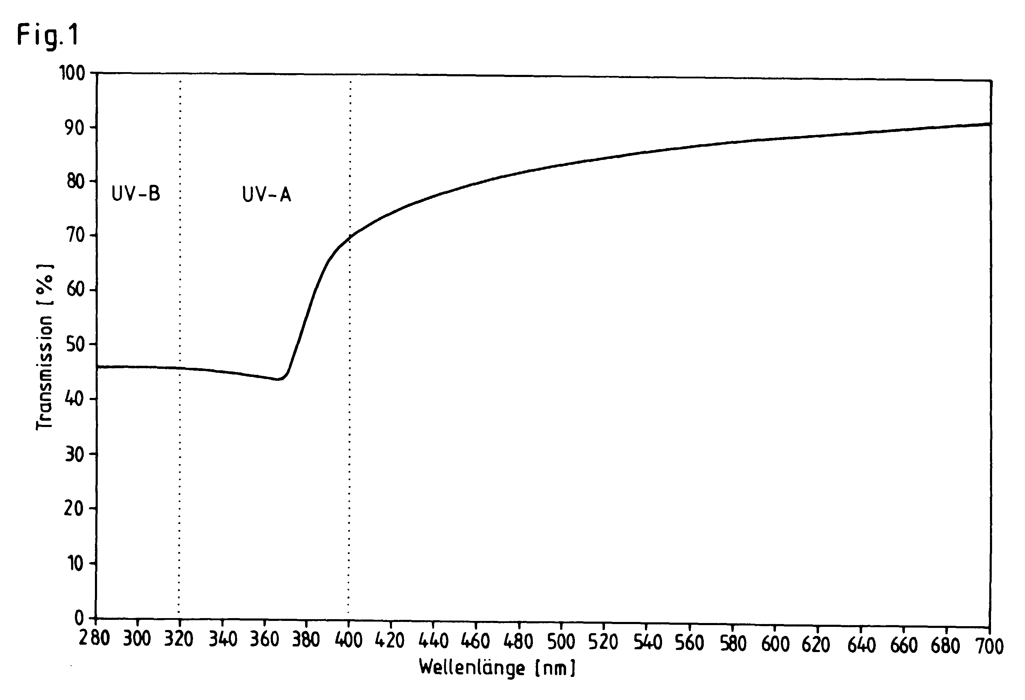

## Patentansprüche

1. Feinteiliges, hochreines, neutrales Zinkoxidpulver für UV-Schutz, dadurch gekennzeichnet, daß es eine spezifische BET-Oberfläche zwischen 30 und 100 m/g, einen pH-Wert zwischen 6,0 und 7,6 sowie einen besonders niedrigen Schwermetallgehalt von jeweils weniger als 5 ppm der Elemente Co, Ni, Pb, Cd, Cu, Mn, Fe und Hg aufweist.

2. Zinkoxidpulver gemäß Anspruch 1, dadurch gekennzeichnet, daß es eine anorganische Nachbehandlungsschicht, bestehend aus einem oder mehreren Hydroxiden, Oxidhydraten oder Oxiden der Elemente Aluminium, Silicium, Zirkonium oder Titan in einer Menge von 0,5 bis 15 Gew.-%, bezogen auf ZnO, aufweist.

3. Zinkoxidpulver gemäß Anspruch 1 oder 2, dadurch gekennzeichnet, daß es hydrophob ist.

4. Zinkoxidpulver gemäß Anspruch 3, dadurch gekennzeichnet, daß es als Hydrophobierungsmittel ein Silikonöl aufweist.

5. Dispersionen von Zinkoxidpulver in einer wäßrigen oder einer Ölphase, die ausgewählt ist aus den Gruppen mineralischer, pflanzlicher oder tierischer Öle oder langkettiger Kohlenwasserstoffverbindungen oder Ester, dadurch gekennzeichnet, daß sie 0,1 bis 50 Gew.-% eines Zinkoxidpulvers gemäß einem oder mehreren der Ansprüche 1 bis 4 enthalten.

6. Sonnenschutz-Zubereitungen und Tageshautpflegepräparate, dadurch gekennzeichnet, daß sie 0.5 bis 20 Gew.-% eines Zinkoxidpulvers gemäß einem oder mehreren der Ansprüche 1 bis 4 entweder allein oder in Kombination mit einem oder mehreren anorganischen und/oder organischen UV-Schutz-Wirkstoffen enthalten.

7. Verfahren zur Herstellung von Zinkoxidpulver gemäß Anspruch 1 durch Fällung von basischem Zinkcarbonat aus gegebenenfalls vorgereinigten Zinksulfat- und/oder Zinkchloridlösungen mittels Alkalicarbonatlösungen, dadurch gekennzeichnet, daß die Fällung diskontinuierlich unter Vorlage der Zinksalzlösung und Zugabe der Alkalicarbonatlösung oder kontinuierlich durch gleichzeitige Zugabe von Zinksalz- und Alkalicarbonatlösung in einem pH-Bereich von 5,2 bis 6,5, vorzugsweise 5,8 bis 6,3, durchgeführt wird, und daß das Fällprodukt von der Mutterlauge abgetrennt, gegebenenfalls gewaschen, kalziniert und gemahlen wird.

8. Verfahren gemäß Anspruch 7, dadurch gekennzeichnet, daß das Filtrat nach der Fällung durch Zugabe weiterer Alkalicarbonatlösung bei einem pH-Wert oberhalb 6,5 aufgearbeitet und das Fällprodukt dieser zweiten Fällung nach Abtrennung von der Mutterlauge und Wiederauflösung in Säure in den Prozeß zurückgeführt wird.

9. Verfahren gemäß Anspruch 7 oder 8, dadurch gekennzeichnet. daß die Fälltemperatur 50 bis 90°C, vorzugsweise 60 bis 80°C, beträgt.

10. Vcrfahren gemäß einem oder mehreren der Ansprüche 7 bis 9, dadurch gekennzeichnet, daß die eingesetzten Lösungen 5 bis 20 Gew.-% Alkalicarbonat und 30 bis 130 g Zink/l enthalten.

11. Verfahren gemäß Anspruch 10, dadurch gekennzeichnet, daß als Alkalicarbonatlösung eine Sodalösung eingesetzt wird.

12. Verfahren gemäß einem oder mehreren der Ansprüche 7 bis 11, dadurch gekennzeichnet, daß das Zinkoxid im Anschluß an die Kalzination einer anorganischen Nachbehandlung unterworfen wird.

13. Verfahren gemäß einem oder mehreren der Ansprüche 7 bis 12, dadurch gekennzeichnet, daß das Zinkoxid im Anschluß an die Kalzination oder im Anschluß an die anorganische Nachbehandlung einer Hydrophobierungsbehandlung unterworfen wird.

14. Verfahren gemäß Anspruch 13, dadurch gekennzeichnet. daß als Hydrophobierungsmittel ein Silikonöl eingesetzt wird.

15. Verwendung von Zinkoxidpulver gemäß einem oder mehreren der Ansprüche 1 bis 4 als UV-Schutz-Komponente in kosmetischen Zubereitungen, insbesondere Sonnenschutz- und Tageshautpflege-Zubereitungen.

16. Verwendung von Zinkoxidpulver gemäß einem oder mehreren der Ansprüche 1 bis 4 als UV-Schutz-Komponente in Kunststoffen und Lacken.

## Claims

1. Finely-divided, highly pure, neutral zinc oxide powder for UV protection, characterised in that it exhibits a BET specific surface area of between 30 and 100 m/g, a pH between 6.0 and 7.6 and a particularly low heavy metal content of less than 5 ppm of each of the elements Co, Ni, Pb, Cd, Cu, Mn, Fe and Hg.

2. Zinc oxide powder according to Claim 1, characterised in that it exhibits an inorganic post-treatment layer comprising one or more hydroxides, hydrated oxides or oxides of the elements aluminium, silicon, zirconium or titanium in a quantity of from 0.5 to 15 wt-%, calculated on ZnO.

3. Zinc oxide powder according to Claim 1 or 2, characterised in that it is hydrophobic.

4. Zinc oxide powder according to Claim 3, characterised in that it exhibits a silicone oil as the hydrophobicity agent.

5. Dispersions of zinc oxide powder in an aqueous or oily phase selected from the groups comprising mineral, vegetable or animal oils or long-chain hydrocarbon compounds or esters, characterised in that they contain from 0.1 to 50 wt-% of a zinc oxide powder according to one or more of Claims 1 to 4.

6. Sunscreen preparations and daytime skin care preparations, characterised in that they contain, either alone or in combination with one or more inorganic and/or organic active substances affording UV protection, from 0.5 to 20 wt-% of a zinc oxide powder according to one or more of Claims 1 to 4.

7. Process for the preparation of zinc oxide powder according to Claim 1 by precipitating basic zinc carbonate from optionally pre-purified zinc sulphate solutions and/or zinc chloride solutions by means of alkali metal carbonate solutions, characterised in that the precipitation is performed in batch-wise manner by taking the zinc salt solution and introducing the alkali metal carbonate solution thereto or in continuous manner by introducing zinc salt solution and alkali metal carbonate solution simultaneously within the pH range 5.2 to 6.5, preferably 5.8 to 6.3, and in that the precipitation product is separated from the mother liquor, is optionally washed, and is calcined and ground.

8. Process according to Claim 7, characterised in that, after the precipitation, the filtrate is worked up by the introduction of further alkali metal carbonate solution at a pH above 6.5, and the product from the latter second precipitation is returned to the process after separation from the mother liquor and redissolution in acid.

9. Process according to Claim 7 or 8, characterised in that the precipitation temperature is from 50 to 90°C, preferably from 60 to 80°C.

10. Process according to one or more of Claims 7 to 9, characterised in that the solutions employed contain from 5 to 20 wt-% alkali metal carbonate and from 30 to 130 g zinc/l.

11. Process according to Claim 10, characterised in that the alkali metal carbonate solution used is a soda solution.

12. Process according to one or more of Claims 7 to 11, characterised in that, following calcining, the zinc oxide undergoes an inorganic post-treatment.

13. Process according to one or more of Claims 7 to 12, characterised in that, following calcining or following the inorganic post-treatment, the zinc oxide undergoes a treatment which renders it hydrophobic.

14. Process according to Claim 13, characterised in that the hydrophobicity agent used is a silicone oil.

15. Use of zinc oxide powder according to one or more of Claims 1 to 4 as a UV protection component in cosmetic preparations, in particular sunscreen and daytime skin care preparations.

16. Use of zinc oxide powder according to one or more of Claims 1 to 4 as a UV protection component in plastics and lacquers.

## Revendications

1. Oxyde de zinc en poudre fine, neutre, à haute pureté, pour protection contre les U.V., caractérisé en ce qu'il a une surface spécifique BET de 30 à 100 m/g, un pH de 6,0 à 7,6, et une teneur particulièrement basse en métaux lourds, de moins de 5 ppm pour chacun des éléments Co, Ni, Pb, Cd, Cu, Mn, Fe et Hg.

2. Oxyde de zinc en poudre selon la revendication 1, caractérisé en ce qu'il porte une couche minérale appliquée par un traitement complémentaire et consistant en un ou plusieurs hydroxydes, oxydes hydratés ou oxydes des éléments aluminium, silicium, zirconium ou titane, en quantité de 0,2 à 15 % du poids du ZnO.

3. Oxyde de zinc en poudre selon la revendication 1 ou 2, caractérisé en ce qu'il est hydrophobe.

4. Oxyde de zinc en poudre selon la revendication 3, caractérisé en ce qu'il porte une huile de silicone en tant qu'agent hydrofugeant.

5. Dispersions d'oxyde de zinc en poudre dans une phase aqueuse ou huileuse choisie parmi les huiles minérales, végétales et animales, les hydrocarbures ou esters à longues chaînes, caractérisées en ce qu'elles contiennent de 0,1 à 50 % en poids d'un oxyde de zinc en poudre selon l'une ou plusieurs des revendications 1 à 4.

6. Produits antisolaires et compositions pour les soins quotidiens de la peau, caractérisés en ce qu'ils contiennent de 0,5 à 20 % en poids d'un oxyde de zinc en poudre selon une ou plusieurs des revendications 1 à 4, seul ou en combinaison avec une ou plusieurs substances minérales et/ou organiques protégeant contre les U.V.

7. Procédé de préparation de l'oxyde de zinc en poudre selon la revendication 1 par précipitation de carbonate de zinc basique à partir de solutions de sulfate de zinc et/ou de chlorure de zinc éventuellement purifiées au préalable, à l'aide de solutions de carbonates alcalins, caractérisé en ce que la précipitation est réalisée en discontinu par addition de la solution de carbonate alcalin à la solution de sel de zinc ou en continu par addition simultanée de la solution de sel de zinc et de la solution de carbonate alcalin dans un intervalle de pH de 5,2 à 6,5, de préférence de 5,8 à 6,3, et en ce que le produit de précipitation est séparé des liqueurs mères, le cas échéant lavé, calciné et broyé.

8. Procédé selon la revendication 7, caractérisé en ce que, après la précipitation, le filtrat est traité par addition de compléments de la solution de carbonate alcalin à un pH supérieur à 6,5, et le produit de cette deuxième précipitation, après séparation des liqueurs mères et redissolution dans un acide, est recyclé dans les opérations.

9. Procédé selon la revendication 7 ou 8, caractérisé en ce que la température de précipitation est de 50 à 90°C, de préférence de 60 à 80°C.

10. Procédé selon l'une ou plusieurs des revendications 7 à 9, caractérisé en ce que les solutions mises en oeuvre contiennent respectivement de 5 à 20 % en poids de carbonate alcalin et de 30 à 130 g de zinc/litre.

11. Procédé selon la revendication 10, caractérisé en ce que la solution de carbonate alcalin utilisée est une solution de carbonate de sodium.

12. Procédé selon l'une ou plusieurs des revendications 7 à 11, caractérisé en ce que, après calcination, l'oxyde de zinc est soumis à un traitement complémentaire minéral.

13. Procédé selon l'une ou plusieurs des revendications 7 à 12, caractérisé en ce que, après la calcination ou après le traitement complémentaire minéral, l'oxyde de zinc est soumis à un traitement d'hydrofugation.

14. Procédé selon la revendication 13, caractérisé en ce que l'on utilise une huile de silicone en tant qu'agent hydrofugeant.

15. Utilisation de l'oxyde de zinc en poudre selon l'une ou plusieurs des revendications 1 à 4 en tant que composant protégeant contre les U.V. dans des produits cosmétiques, en particulier des produits antisolaires et des produits pour les traitements quotidiens de la peau.

16. Utilisation de l'oxyde de zinc en poudre selon l'une ou plusieurs des revendications 1 à 4 en tant que composant protégeant contre les U.V. dans des résines synthétiques et des peintures.
